(19) **Europäisches Patentamt**

**European Patent Office**

**Office européen des brevets**

(11) **EP 1 173 739 B1**

(12) **EUROPÄISCHE PATENTSCHRIFT**

(45) Veröffentlichungstag und Bekanntmachung des Hinweises auf die Patenterteilung:
**05.03.2003 Patentblatt 2003/10**

(21) Anmeldenummer: **00934958.0**

(22) Anmeldetag: **22.04.2000**

(51) Int Cl.$^7$: **G01L 9/00**

(86) Internationale Anmeldenummer:
**PCT/EP00/03654**

(87) Internationale Veröffentlichungsnummer:
**WO 00/065322 (02.11.2000 Gazette 2000/44)**

(54) **VERFAHREN ZUR NICHTINVASIVEN INNENDRUCKMESSUNG**

METHOD FOR THE NON-INVASIVE MEASUREMENT OF AN INTERNAL PRESSURE

PROCEDE POUR MESURER UNE PRESSION INTERNE DE MANIERE NON INVASIVE

(84) Benannte Vertragsstaaten:
**AT BE CH CY DE DK ES FI FR GB GR IE IT LI LU MC NL PT SE**

(30) Priorität: **26.04.1999 DE 19918714**

(43) Veröffentlichungstag der Anmeldung:
**23.01.2002 Patentblatt 2002/04**

(73) Patentinhaber:
• **Silber, Gerhard**
  **60435 Frankfurt/Main (DE)**
• **Stanull, Michael**
  **63179 Obertshausen (DE)**
• **Wackenreuther, Matthias**
  **55291 Saulheim (DE)**
• **Schüttler, Eva-Maria**
  **60435 Frankfurt/Main (DE)**
• **Lohberg, Jochen**
  **61206 Wöllstadt (DE)**

(72) Erfinder:
• **Silber, Gerhard**
  **60435 Frankfurt/Main (DE)**
• **Stanull, Michael**
  **63179 Obertshausen (DE)**
• **Wackenreuther, Matthias**
  **55291 Saulheim (DE)**
• **Schüttler, Eva-Maria**
  **60435 Frankfurt/Main (DE)**
• **Lohberg, Jochen**
  **61206 Wöllstadt (DE)**

(74) Vertreter: **Knoblauch, Andreas, Dr.-Ing.**
  **Schlosserstrasse 23**
  **60322 Frankfurt (DE)**

(56) Entgegenhaltungen:
**EP-A- 0 501 234     DE-A- 19 747 254**

**Beschreibung**

Die Erfindung betrifft ein Verfahren zur nichtinvasiven Innendruckmessung in elastischen Gefäßen, bei dem eine Kraft an der Mantelfläche des Gefäßes gemessen und der Innendruck mit Hilfe einer Differenz aus der gemessenen Kraft und einem im voraus abgeschätzten Relaxationsverlauf des Gefäßes ermittelt wird.

In einer Reihe von Anwendungsfällen möchte man den Innendruck in einem Schlauch oder einem anderen Gefäß ermitteln, ohne daß man eine Verbindung zum Inneren des Schlauches herstellen muß. Dies gilt insbesondere im medizinischen Bereich, wo man die Gefahr einer Infektion des Patienten dadurch klein halten möchte, daß man Keimen möglichst wenig Zutrittsöffnungen schafft. Anwendungsbeispiele sind Blutwäsche bei Dialyse-Patienten oder der Anschluß einer Herz-Lungen-Maschine.

Es ist nun bekannt, daß viele der Materialien, die für die Gefäße, insbesondere für Schläuche, verwendet werden, ein Kriechverhalten haben, so daß auch bei konstantem Innendruck mit der Zeit eine Veränderung der gemessenen Kraft auftritt. Dies täuscht den Abfall des Innendrucks im Schlauch vor.

Es ist daher in EP 0 501 234 B1 vorgeschlagen worden, der eigentlichen Meßzeit eine Vorbereitungszeit vorzuschalten, in der der Schlauch über einen längeren Zeitraum verformend vorgespannt wird. Man nimmt dabei an, daß nach dieser Zeit keine Kriechvorgänge mehr auftreten und das ermittelte Signal, nämlich die Reaktionskraft, eine zutreffende Aussage über den tatsächlich im Schlauch herrschenden Innendruck gibt.

Eine verbesserte Messung ergibt sich bei einem Verfahren, das in der nachveröffentlichten DE 197 47 254 A1 beschrieben ist. Hier geht man davon aus, daß das Material des Gefäßes auch nach einer gewissen Zeit noch kriechen wird. Man berücksichtigt dieses Verhalten, das auch Relaxationsverhalten genannt wird, durch eine Funktion, für die die nötigen Parameter vor der Messung ermittelt werden. Bei der Messung berücksichtigt man dann die Differenz zwischen den gemessenen Werten und der mit Hilfe der Parameter vorhergesagten oder im voraus abgeschätzten Relaxationsfunktion, um den eigentlichen Innendruck zu errechnen.

Der Erfindung liegt die Aufgabe zugrunde, bei einfacher Innendruckmessung eine gute Genauigkeit zu erzielen.

Diese Aufgabe wird bei einem Verfahren der eingangs genannten Art dadurch gelöst, daß der Relaxationsverlauf nach Beginn der Messung wiederholt überprüft wird.

Mit der Relaxationsfunktion macht man im Grunde eine Vorhersage über das künftige Verhalten des Gefäßes. Erfindungsgemäß wird nun nach Beginn der Messung überprüft, ob die Vorhersage zutrifft oder nicht. Im letzten Fall wird die Vorhersage korrigiert, so daß man das Relaxationsverhalten des Gefäßes mit einer höheren Zuverlässigkeit vorhersagen kann. Bei der weiteren Messung kann man dann davon ausgehen, daß die Differenz zwischen dem Relaxationsverlauf des Gefäßes und der gemessenen Kraft um so genauer ist, je kürzer die letzte Vorhersage des Relaxationsverlaufs zurückliegt. Hierbei kommt hinzu, daß im Laufe der Zeit immer mehr Meßwerte zur Verfügung stehen. Je mehr Meßwerte zur Verfügung stehen, desto genauer kann man den Relaxationsverlauf nachbilden. Je genauer die Nachbildung ist, desto größer ist die Wahrscheinlichkeit, daß zumindest für die nähere Zukunft die Vorhersage "stimmt". Damit läßt sich auf einfache Art und Weise eine höhere Genauigkeit bei der Innendruckmessung erzielen. Da die Meßwerte der Kraft ohnehin zur Verfügung stehen, ist lediglich ein geringfügig höherer Aufwand bei der Verarbeitung der Meßwerte erforderlich. Dieser Aufwand läßt sich aber mit heute zur Verfügung stehenden Prozessuren leicht bewältigen.

Vorzugsweise wird der Relaxationsverlauf mit Hilfe eines Mittelungsverfahrens ermittelt. Dies ist insbesondere dann von Vorteil, wenn der Innendruck selbst pulsiert oder sich annähernd periodisch verändert, wie es beispielsweise bei der Verwendung von peristaltischen Pumpen oder Kolbenpumpen zur Förderung eines Fluids durch das Gefäß der Fall ist. Dann wird die Erfassung der Relaxation aufgrund der Pulsation zwar nicht unmöglich, aber schwierig. Diese Schwierigkeit kann man auf einfache Art und Weise dadurch umgehen, daß man über eine vorbestimmte Zeit den Mittelwert oder einen Durchschnittswert der gemessenen Kräfte bildet oder die gemessenen Werte filtert. Der Zeitraum, in dem der Mittelwert gebildet wird, wird als Zeitfenster mitgeführt. Der Mittelwert bezieht sich also immer auch auf einen Zeitraum mit vorbestimmter Länge vor dem aktuellen Zeitpunkt.

Vorzugsweise erfolgt eine Mittelwertbildung auf mindestens zwei unterschiedliche Arten, die sich durch ihre Glättungsbreiten unterscheiden. Beispielsweise verwendet man für die eine Mittelwertbildung einen Zeitraum, der doppelt so lang ist wie der für die andere Mittelwertbildung. Damit bekommt man eine verbesserte Kontrolle und kann vor allem Fehler und Störungen schneller erkennen.

Dies gilt insbesondere dann, wenn fortlaufend eine Differenz der Mittelwerte mit unterschiedlicher Glättungsbreite gebildet wird. Ein Mittelwert, der über einen größeren Zeitraum gebildet wird, reagiert träger auf eine Änderung des Verhaltens als ein Mittelwert, der über einen kürzeren Zeitraum gebildet wird. Wenn man unterstellt, daß die Messung der Kräfte in beiden Fällen mit der gleichen zeitlichen Auflösung erfolgt, dann kann man auch davon ausgehen, daß bei einer größeren Anzahl von Meßwerten der Mittelwert dem eigentlichen Verlauf mit einer größeren Trägheit folgt als bei einer kleineren Anzahl von Meßwerten. Im "ungestörten" Fall spielt die Trägheit keine Rolle. Die Mittelwerte werden also weitgehend übereinstimmen. Die Unterschiede liegen dann nur in einem zulässigen Toleranzbereich. Wenn jedoch der Innendruck stark ansteigt, beispielsweise in Form eines "Sprunges", dann werden sich die beiden Mittelwerte mit unterschiedlicher Glättungsbreite sehr stark unterscheiden. Anhand dieser Differenz kann man einen derartigen Sprung dann erkennen.

Vorzugsweise wird eine Periodizität der gemessenen Kraft ermittelt und eine Fensterbreite der Mittelwertbildung wird zumindest von Zeit zu Zeit auf die Periodizität abgestimmt. Die Periodizität kann man beispielsweise ermitteln, indem man die Minima über einen gewissen Zeitraum zählt. Man kann dann dafür sorgen, daß die Mittelwertbildung aus einer vorbestimmten Anzahl von ganzen Perioden erfolgt. Dies verbessert die Genauigkeit der Mittelwerte. Da sich die Periodizität unter Umständen ändern kann, kann man beispielsweise vorsehen, daß man eine vorbestimmte Anzahl von Mittelwertbildungen mit gleicher Glättungslänge vornimmt und dann die Periodizität neu bestimmt.

In einer bevorzugten Ausgestaltung ist vorgesehen, daß man fortlaufend eine erste Grenze bildet, die sich daraus ergibt, daß der Relaxationsverlauf monoton fällt, und eine zweite Grenze, die sich daraus ergibt, daß die Steigung des Relaxationsverlaufs abnimmt, und eine Veränderung des Innendrucks erkannt wird, wenn der Relaxationsverlauf eine der beiden Grenzen überschreitet. Die Änderung der gemessenen Kräfte kann zwei Ursachen haben. Zum einen ändern sich die Kräfte aufgrund des Relaxationsverhaltens des Gefäßes. Zum anderen ändern sich die gemessenen Kräfte dann, wenn sich der Innendruck ändert. Diese Änderung kann auf verschiedene Arten erfolgen. Sie kann beispielsweise sprungartig sein. Diese Änderung wird durch die Differenzbildung von Mittelwerten mit unterschiedlicher Glättungsbreite recht zuverlässig erkannt. Die Änderung kann aber auch durch einen sich langsam ändernden Innendruck erfolgen, beispielsweise dann, wenn sich eine Infusionsnadel langsam zusetzt. Diese Änderung kann durchaus so klein sein, daß sie die durch das Relaxationsverhalten bedingten Änderungen der Meßwerte nur geringfügig überschreitet. Derartige Änderungen kann man aber dadurch erkennen, daß man ein "Steigungsdreieck" in den Relaxationsverlauf legt und überprüft, ob die gemessenen Werte noch in diesem Dreieck liegen. Man weiß nämlich, daß der Relaxationsverlauf monoton fällt. Wenn also Meßwerte, genauer gesagt, der Mittelwert aus den Meßwerten, ansteigt, dann kann dies nicht mit der Relaxation zu erklären sein. Umgekehrt ist bekannt, daß die negative Steigung des Relaxationsverlaufs betragsmäßig immer kleiner wird, sich also asymptotisch einer Geraden annähert. Wenn dieses Gefälle nun auf einmal größer wird, dann kann dies auch nicht am Relaxationsverhalten des Gefäßes liegen, sondern deutet auf eine Innendruckveränderung hin. Unter Berücksichtigung dieser Erkenntnisse kann man dann die Verarbeitung der gemessenen Kräfte entsprechend steuern.

Zur Vorhersage des Relaxationsverlaufs bestimmt man vorzugsweise wiederholt Stützstellen. Die Verarbeitung von einzelnen Werten, nämlich den Werten an den Stützstellen, ist wesentlich einfacher als die Verarbeitung einer fortlaufenden Funktion mit theoretisch unendlich vielen Werten. Es hat sich herausgestellt, daß man auch mit einer bestimmten Anzahl von Stützstellen die nötigen Informationen gewinnen kann, um den Relaxationsverlauf zuverlässig genug vorhersagen zu können.

Vorzugsweise werden die Stützstellen in einer Initialisierungsphase an vorgegebenen Zeitpunkten und in einer Meßphase nach einer vorbestimmten Änderung des vorhergesagten Relaxationsverlaufs ermittelt. In der Initialisierungsphase liegt beispielsweise Atmosphärendruck im Innern des Gefäßes an. Man kann nun in einem relativ kurzen Zeitpunkt eine ausreichende Anzahl von Meßwerten an den Stützstellen ermitteln, weil diese Stützstellen zeitlich festgelegt sind. Mit den ermittelten Meßwerten läßt sich der Relaxationsverlauf zumindest für die nähere Zukunft vorhersagen. Beispielsweise reichen bereits vier Stützstellen aus, um eine erste Vorhersage treffen zu können. Mit weiteren sechs Stützstellen läßt sich dann die Vorhersage so stabilisieren, daß mit der Messung begonnen werden kann. Mit zunehmender Zeitdauer werden allerdings die relaxationsbedingten Unterschiede der Meßwerte (bei der Messung geht man zur Ermittlung des Relaxationsverlaufes von den eigentlichen Meßwerten ab und verwendet statt dessen Mittelwerte) immer kleiner, so daß aufgrund von Meßungenauigkeiten die Gefahr einer Verfälschung der Ermittlung besteht. Man wartet daher ab, bis davon auszugehen ist, daß der Relaxationsverlauf um einen Wert abgenommen ist, der mit ausreichender Genauigkeit ermittelt werden kann. Die nächste Stützstelle wird dann erst zu diesem Zeitpunkt vorgesehen.

Vorzugsweise werden Stützstellen nicht ermittelt, solange eine Änderung des Innendrucks erkannt wird. Dies kann beispielsweise dann der Fall sein, wenn zumindest ein Mittelwert einen Verlauf mit einer Steigerung aufweist, die ein vorbestimmtes Maß übersteigt. In derartigen Rampenabschnitten ist davon auszugehen, daß sich den Änderungen der Meßwerte bzw. der Mittelwerte, die durch das Relaxationsverhalten des Gefäßes bedingt sind, Änderungen überlagert, die durch den Innendruck bedingt sind. Da eine saubere Trennung dieser beiden Einflußfaktoren in der Regel nicht oder nur unter Schwierigkeiten möglich ist, verzichtet man in diesen Zeitabschnitten auf die Bildung von Stützstellen.

Vorzugsweise wird der Relaxationsverlauf anhand der Stützstellen mit Hilfe eines nichtlinearen Optimierungsverfahrens vorhergesagt. Derartige Verfahren sind an und für sich bekannt. Beispielsweise kann man die Evolutionsstrategie, das Simulated Annealing, das Treshholding Accept, das Randomcost-Verfahren und das Self Adapted Annealing verwenden. Diese Verfahren erlauben es, aus der jüngeren Vergangenheit eine Vorhersage für die nähere Zukunft zu treffen.

Vorzugsweise erfolgt die Vorhersage in der Initialisierungsphase stützstellengesteuert und in der Meßphase zeitgesteuert. Damit erreicht man in der Initialisierungsphase eine relativ schnelle Vorhersage. In der Meßphase wird die Vorhersage nach vorbestimmten Zeitabständen wiederholt. Dies reicht in der Regel aus. Allerdings kann es hierbei vorkommen, daß aufeinanderfolgende Vorhersagen auf den gleichen Stützstellen basieren, weil zwischenzeitlich keine neue Stützstelle eingerichtet worden ist.

Vorteilhafterweise wird zur Optimierung eine vorbestimmte Anzahl der zuletzt ermittelten Stützstellen verwendet. Man verwendet also beispielsweise immer die an den letzten zwanzig Stützstellen ermittelten Werte. Werte, die zu vorherliegenden Stützstellen gehören, kann man in der Regel verwerfen, weil ihr Einfluß auf die Vorhersage für die nähere Zukunft zu gering ist.

Vorzugsweise wird der Relaxationsverlauf anhand der Stützstellen mit Hilfe eines mathematischen Modells des Schlauches vorhergesagt, beispielsweise eines Abel-Kerns. Die Optimierung wird dann einfacher und zuverlässiger.

Die Erfindung wird im folgenden anhand eines bevorzugten Ausführungsbeispiels in Verbindung mit der Zeichnung beschrieben. Hierin zeigen:

Fig. 1    einen Relaxationsverlauf mit Stützstellen und Drucksprüngen,

Fig. 2    einen vergrößerten Ausschnitt des Verlaufs aus Fig. 1,

Fig. 3    einen Verlauf von Meßwerten mit Sprüngen,

Fig. 4    eine Darstellung der Differenzen von Mittelwerten mit unterschiedlichen Glättungsbreiten und

Fig. 5    eine schematische Darstellung einer Meßanordnung.

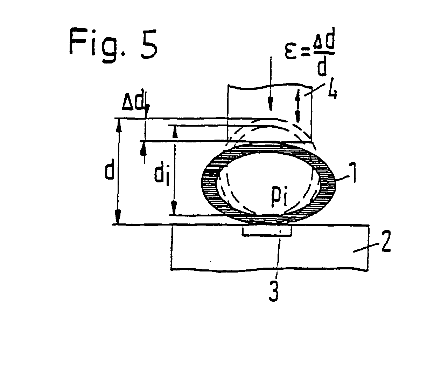

Zur nichtinvasiven Schlauchdruckmessung, d. h. zur nichtinvasiven Permanent-Innendruckbestimmung in elastischen Gefäßen, wie Rohren, Schläuchen etc., wird eine in Fig. 5 schematisch dargestellte Vorrichtung verwendet. Einzelheiten hierzu sind in DE 197 47 254 A1 beschrieben. Die Innendruckbestimmung erfolgt mit Hilfe einer Kraft- oder Druckmessung an der Außenwand des Gefäßes 1. Nichtinvasiv bedeutet hier, daß die Gefäßoberfläche weder verändert werden muß, noch eine Verbindung zwischen dem Gefäßinneren und der Meßsensorik erforderlich ist, etwa in Form einer T-Abzweigung. Zu fördernde Medien können Flüssigkeiten und Gase sein, allgemein Fluide. Als Mittel zur Erzeugung des Drucks können beispielsweise Rollerpumpen, peristaltische Pumpen oder Zentrifugalpumpen verwendet werden. Das Meßprinzip kann überall dort angewendet werden, wo eine Verbindung oder ein Kontakt zwischen dem geförderten Fluid und der Umgebung unerwünscht oder gefährlich ist. Besonders bevorzugte Anwendungsgebiete sind solche, wo eine Kontaminierung des Fluids bzw. eine Infektionsgefahr der Außenwelt, beispielsweise medizinisches Pflegepersonal, auszuschließen ist. Verwendet werden kann das Meßverfahren beispielsweise in der Hämodialyse, in der Infusionstechnik, bei Herz-Lungen-Maschinen, in der Lebensmitteltechnologie oder in der allgemeinen Verfahrenstechnik.

Als Beispiel für das Gefäß wird ein Schlauch 1 verwendet, der in unverformtem Zustand gestrichelt und in verformtem Zustand dick ausgezogen und schraffiert dargestellt ist. Der Schlauch 1 liegt auf einer Auflage 2 auf, die im Bereich des Kontakts mit dem Schlauch 1 einen Kraftsensor 3 aufweist. Ein Stempel 4 wirkt auf den Schlauch 1 und verformt ihn, indem der Stempel über eine Strecke Δd in Richtung auf die Auflage 2 bewegt wird. Ausgangspunkt für die Strecke Δd ist der Außendurchmesser d des Schlauches 1 in unverformtem Zustand. Die Zustellbewegung des Stempels 4 kann normiert werden auf

$$\varepsilon = \frac{\Delta d}{d}$$

Im Schlauch 1 herrscht ein Druck Pi.
Die Kombination des Innendrucks Pi mit der Verformung des Schlauchs 1 durch den Stempel 4 führt zu einer Kraft oder einer Reaktionskraft, die man am Kraftsensor 3 ermitteln kann. Selbstverständlich ist es auch möglich, den Kraftsensor in der Stirnfläche des Stempels 4 unterzubringen. Die Zustellbewegung ε ist hier übertrieben groß dargestellt. Im Grunde ist lediglich eine Bewegung erforderlich, die ausreicht, um bei den auftretenden Drücken Pi im Innern des Schlauches 1 eine meßbare Kraft am Sensor 3 zu erzeugen.

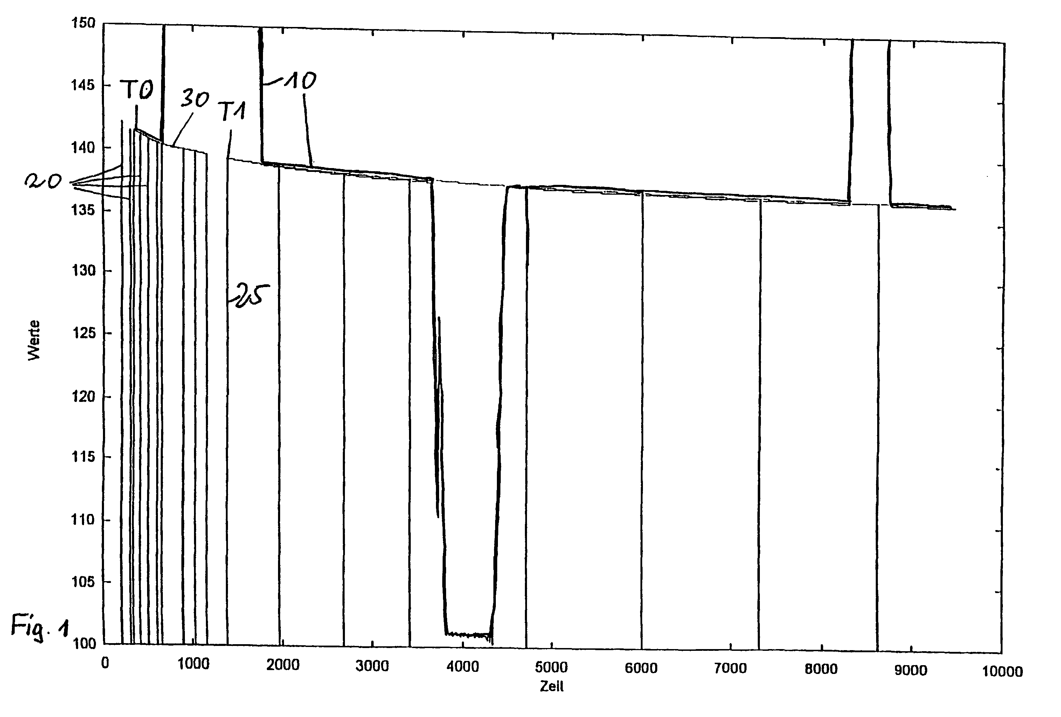

Fig. 1 zeigt nun mit einer relativ dicken Linie 10 die vom Sensor 3 ermittelten Kraftwerte. Um diese Kraftwerte verfolgen zu können, auch wenn der Innendruck Pi pulsiert, wird aus den gemessenen Kraftwerten der Mittelwert gebildet, d.h. ausgehend vom aktuellen Zeitpunkt werden die einzelnen Kraftwerte über einen vorher festgelegten zurückliegenden Zeitraum aufsummiert und durch den Zeitraum dividiert. Dementsprechend ergibt sich auch bei einem pulsierenden Innendruck, dessen Pulsationsamplitude aber im wesentlichen konstant ist, eine relativ glatte Kurve 10. Allerdings sind in dieser Kurve 10 Sprünge erkennbar, an denen sich das Druckniveau deutlich ändert.
Für die nachfolgende Erläuterung wird angenommen, daß ein von Atmosphärendruck verschiedener Druck nur in den Zeitbereichen anliegt, in denen die Sprünge erkennbar sind. Die übrige Änderung des Mittelwertes ist durch die Relaxation des Schlauches 1 bedingt. Die Ermittlung des eigentlichen Drucks erfolgt dann mit Hilfe einer Differenz aus den gemessenen Kraftwerten und der durch die Relaxationskurve gebildeten "Null-Linie".

Um diese Kurve zu ermitteln, d.h. den Relaxationsverlauf, wird der Innendruck Pi des Schlauches 1 zunächst auf den Atmosphärendruck gesetzt und durch Zustellung des Stempels 4 eine vorbestimmte Kraft am Sensor 3 erzeugt und dort gemessen. Diese Kraft wird in kurz aufeinanderfolgenden Zeitpunkten an sogenannten Stützstellen 20 gemessen. In der Initialisierungsphase, die der eigentlichen Messung vorausgeht, können beispielsweise vier Stützstellen festgelegt sein. Anhand des Relaxationsverlaufes an diesen vier Stützstellen 20 kann man nun den weiteren Relaxationsverlauf vorhersagen, der durch eine Kurve 30 dargestellt ist. Zur Vorhersage des Relaxationsverlaufes 30 kann man im Grunde jedes nichtlineare Optimierungsverfahren verwenden. Erfolgreich getestet wurden beispielsweise die Evolutionsstrategie, das Simulated Annealing, das Treshholding Accept, das Randomcost-Verfahren und das Self Adapted Annealing.

Sobald der Relaxationsverlauf 30 vorhergesagt werden kann, kann man zu jedem Zeitpunkt die benötigte Differenz aus den gemessenen Werten und dem vorhergesagten Relaxationsverlauf bilden.

Aus Gründen der Deutlichkeit wurde in Fig. 1 ab dem Zeitpunkt 2000 (horizontale Achse) der Verlauf 10 der Mittelwerte etwas über dem Relaxationsverlauf 30 gezeigt. In Wirklichkeit stimmen aber außerhalb der Sprünge die beiden Verläufe 10 und 30 überein, würden also aufeinanderliegen.

Die bis zum Zeitpunkt T0 bestimmte Vorhersage ist lediglich eine grobe Wiedergabe des Relaxationsverhaltens, welche zum Zeitpunkt T1 stabilisiert wird.

Auch nach Beginn der Messung zum Zeitpunkt T1 werden weitere Stützstellen 25 gebildet und der vorhergesagte Relaxationsverlauf wird anhand dieser Stützstellen überprüft.

Hierbei ist zu bemerken, daß die Stützstellen in der Initialisierungsphase auf festgelegten Zeitpunkten liegen. Nach der Initialisierungsphase werden Stützstellen nur dort etabliert, wo aufgrund des vorhergesagten Relaxationsverlaufes anzunehmen ist, daß die Relaxationswerte an diesen Stützstellen eine Differenz haben, die groß genug ist, um mit ausreichender Zuverlässigkeit gemessen zu werden.

Es ist daher erkennbar, daß die Abstände zwischen einzelnen Stützstellen immer größer werden.

Man ermittelt also zunächst die Kurve 10 aus den Mittelwerten über ein vorbestimmtes Zeitfenster. Aus dieser Kurve 10 ermittelt man an den Stützstellen 20, 25. Für die Vorhersage des Relaxationsverlaufes oder Trends verwendet man dann die Werte an den letzten zwanzig Stützstellen. Falls noch keine zwanzig Stützstellen vorhanden sind, verwendet man eben alle zurückliegenden Stützstellen.

Wie bereits erläutert worden ist, ist es in manchen Fällen schwierig zu erkennen, ob eine Änderung der Meßwerte, die vom Sensor 3 aufgenommen werden, auf eine Änderung des Innendrucks Pi oder auf das Relaxationsverhalten des Schlauches 1 zurückzuführen ist.

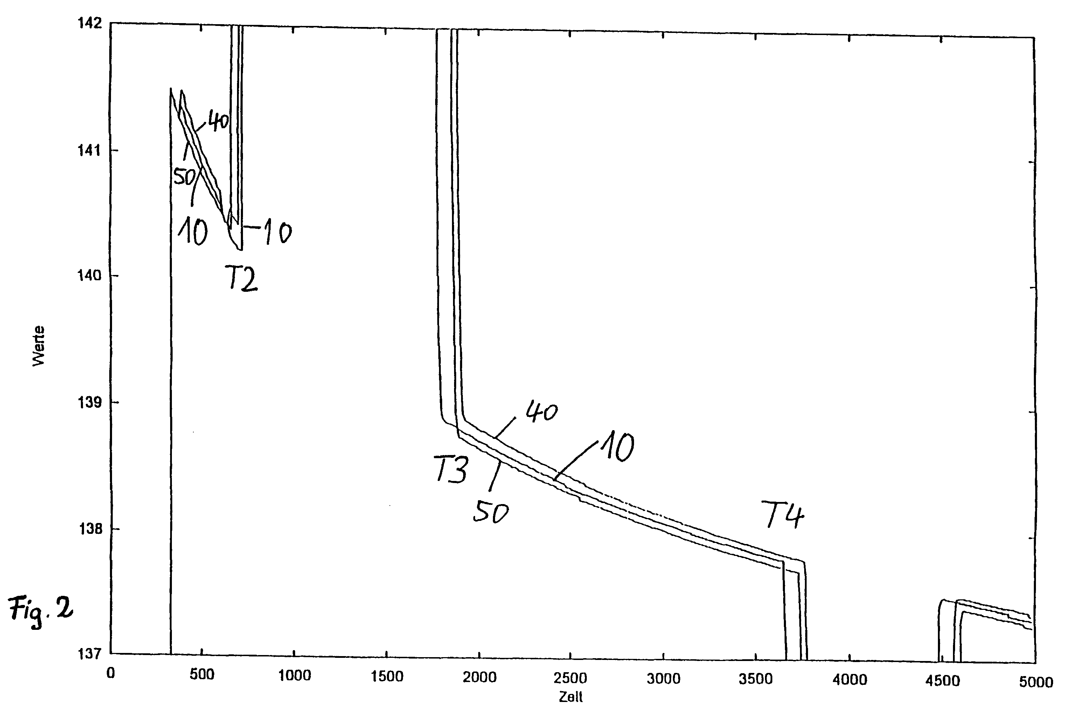

Eine Möglichkeit, um hierüber eine Entscheidung zu treffen, ist in Fig. 2 offenbart. Fig. 2 zeigt einen vergrößerten Ausschnitt aus Fig. 1. Eingezeichnet ist hier die Linie 10, die die Mittelwerte darstellt. Die Mittelwerte werden auch als bewegter Mittelwert oder gleitender Mittelwert bezeichnet, weil diese Mittelwerte immer über ein Zeitfenster vorbestimmter Länge in die Vergangenheit zurückgemittelt werden.

Man bildet nun zwei Grenzen, die durch Linien 40, 50 dargestellt sind. Die erste Grenze 40 beruht auf der Tatsache, daß der Relaxationsverlauf monoton fällt.

Jegliches Ansteigen der Mittelwerte 10 kann dann nicht auf die Relaxation zurückzuführen sein, sondern muß auf einer Veränderung des Innendrucks beruhen. Natürlich wird man hier einen gewissen Abstand einhalten, weil die Mittelwerte einen gewissen Streubereich haben, der durch Meßungenauigkeiten verursacht wird.

Die andere Grenze 50 wird aufgrund der Voraussetzung festgelegt, daß der Betrag der Steigung des Relaxationsverlaufes immer abnimmt. Das Gefälle wird also im Laufe der Zeit immer schwächer. Wenn sich die Mittelwerte 10 stärker abschwächen, als es die Grenze 50 erlaubt, dann liegt ebenfalls eine Veränderung des Innendrucks Pi vor. Man kann in Fig. 2 erkennen, daß die Kurve 10 der Mittelwerte zu einem Zeitpunkt T2 den Bereich zwischen den beiden Grenzen 40, 50 verlassen hat. Dies muß dann auf einen Drucksprung zurückzuführen sein. Zu einem Zeitpunkt T3 tritt die Kurve 10 der Mittelwerte wieder in den Bereich zwischen den beiden Grenzen 40, 50 ein. Ab diesem Zeitpunkt T3 kann man mit guter Näherung davon ausgehen, daß Änderungen in den Mittelwerten 10 durch die Relaxation des Schlauches 1 bedingt sind. Zu einem Zeitpunkt T4 tritt wieder ein Drucksprung auf, der dadurch festgestellt wird, daß die Kurve 10 den Bereich zwischen den Grenzen 40, 50 verläßt.

Mit dieser Überprüfungsmöglichkeit kann man aber nicht nur Sprünge ermitteln, sondern auch langsame Druckänderungen, die sich beispielsweise dadurch ergeben können, daß eine Infusionsnadel sich im Laufe der Zeit zusetzt.

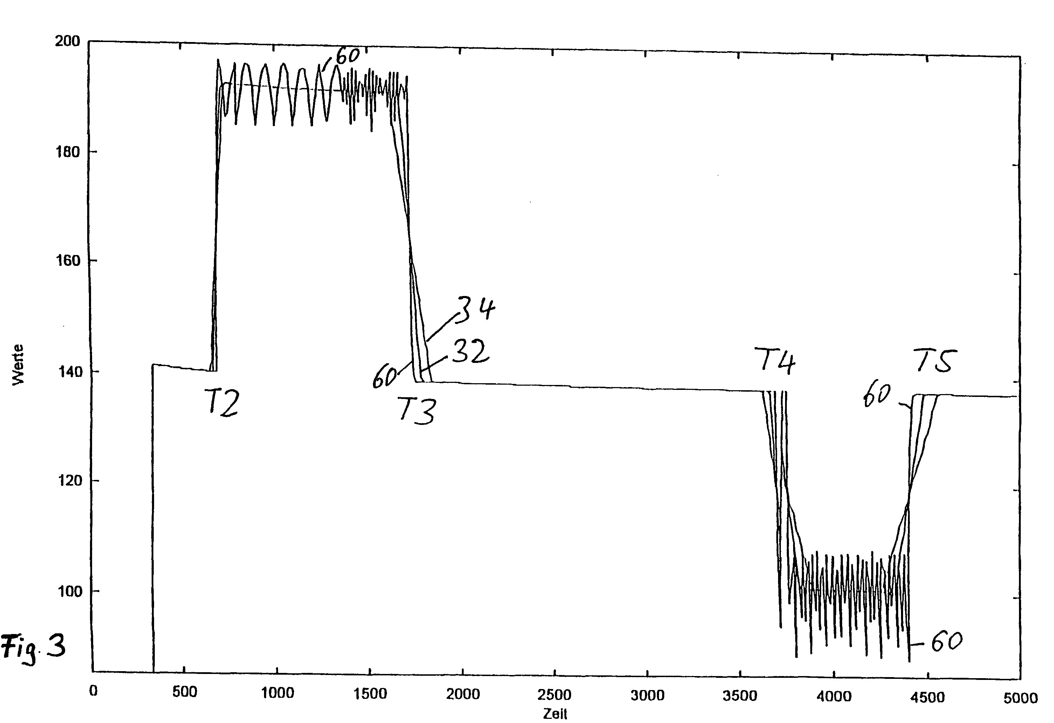

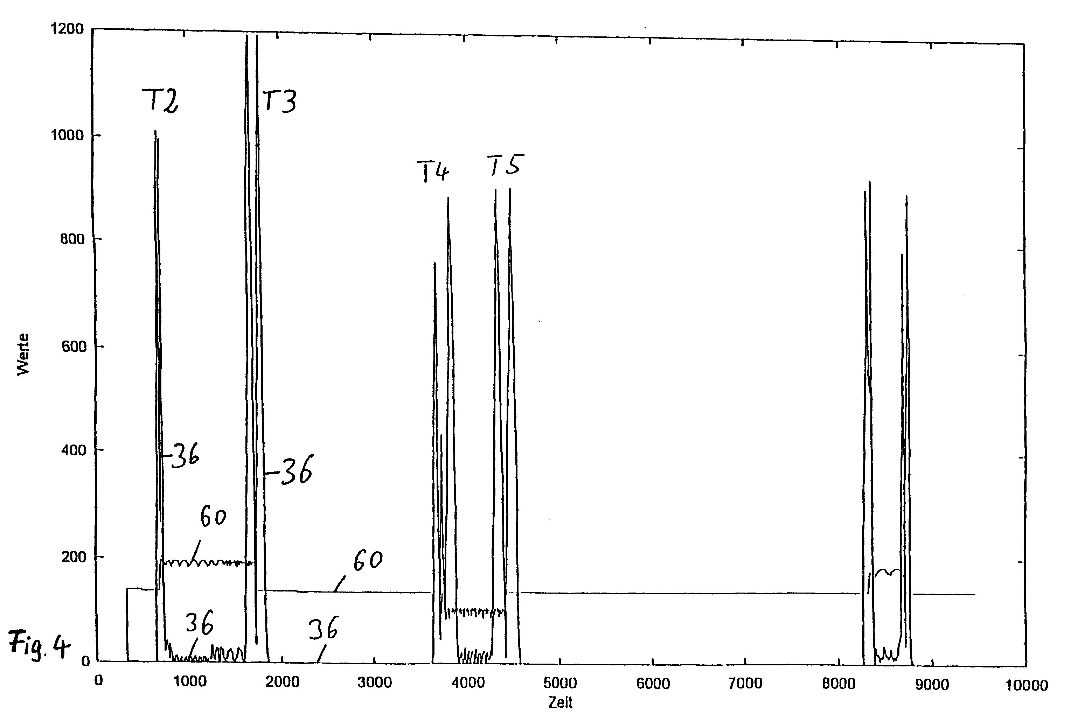

Eine weitere Möglichkeit, um Sprünge im Druckverlauf zu entdecken, zeigen die Fig. 3 und 4. Die Sprünge sind ein besonders kritisches Kriterium.

Fig. 3 zeigt eine Kurve 60 der Meßwerte, d.h. der am Sensor 3 tatsächlich ermittelten Kraftwerte. Es ist zu erkennen, daß die Kraft zwischen den Zeitpunkten T2 und T3 positiv auf eine höhere Amplitude springt und dort schwingt, während sie zwischen den Zeitpunkten T4 und T5 gegenüber der vorhergesagten Relaxation auf einen negativen Wert springt.

Man bildet nun zwei Mittelwerte 32, 34. Der Mittelwert 32 wird beispielsweise über die letzten fünfzig Abtastwerte gebildet, der Mittelwert 34 über die letzten hundert Abtastwerte. Beide Mittelwerte 32, 34 werden selbstverständlich fortlaufend gebildet und bei jedem neuen Abtastwert aktualisiert. Dementsprechend reagiert der Mittelwert 32, der eine geringere Glättungsbreite hat als der Mittelwert 34, schneller auf Änderungen des Meßsignals 60, als der Mittelwert 34. Dies ist in Fig. 3 bereits klar erkennbar. Noch deutlicher wird die Situation allerdings, wenn man sich Fig. 4 betrachtet. In Fig. 4 ist die Differenz der Mittelwerte als eine Kurve 36 aufgetragen. Zusätzlich ist die Kurve 60 der Meßsignale eingezeichnet.

Es läßt sich klar erkennen, daß die Kurve 36 der Differenzen der Mittelwerte 32, 34 normalerweise im Bereich der Null-Linie liegt. Dort, wo die Meßsignale 60 pulsieren, also zwischen den Zeitpunkten T2 und T3 bzw. T4 und T5, pulsiert auch die Differenz 36.

An den "Sprungstellen", also an den Zeitpunkten T2-T5, steigen die Differenzen 36 allerdings ausgesprochen stark an, so daß Sprünge des Innendrucks Pi klar erkennbar sind.

Zur Auswertung der Meßwerte vom Sensor 3 wird also zunächst der Relaxationsverlauf 30 vorhergesagt. Die notwendigen Informationen hierzu erhält man aus den Werten an den Stützstellen 20 in der Initialisierungsphase. Wenn ein Sprung auftritt, dann wird die gemessene Amplitude um den vorhergesagten Wert des Relaxationsverlaufs vermindert. Aus dieser Differenz kann man dann den eigentlichen Innendruck-Wert errechnen. Wie aus Fig. 3 erkennbar ist, unterliegen auch die erhöhten Meßwerte zwischen den Zeitpunkten T2 und T3 einer gewissen Abnahme, die auf die Relaxation zurückzuführen ist. Da die "Sprunghöhe" am Anfang bekannt ist, kann man nun auch weitere Stützstellen 25 (Fig. 1) verwenden, um den Relaxationsverlauf 30 erneut vorherzusagen. Hierzu legt man die beispielsweise an den letzten zwanzig Stützstellen ermittelten Werte in einem Schieberegister ab und verwendet diese zwanzig Werte in einem der oben genannten nichtlinearen Optimierungsverfahren, um den Relaxationsverlauf 30 oder den Trend für die nähere Zukunft vorherzusagen. Da auf diese Weise der Relaxationsverlauf fortlaufend überprüft und korrigiert werden kann, erhält man auch bei länger anhaltenden Messungen stets ein zuverlässiges Meßergebnis, das die Relaxation des Schlauches 1 berücksichtigt.

Das Verfahren läßt sich also kurz wie folgt zusammenfassen:

Es werden kontinuierlich Meßsignale aufgenommen und Stützstellen gespeichert. Man ermittelt Rampen und schleichende Druckanstiege mit Hilfe der Mittelwerte und/oder des Steigungsdreiecks. Das mathematische Modell wird mit Hilfe eines nichtlinearen Optimierungsverfahrens zur Vorhersage der Relaxation anhand der letzten Stützstellen angepaßt. Die Anpassung wird zyklisch wiederholt und verbessert. Stützstellen werden nicht generiert, wenn Innendruckänderungen erkannt werden, beispielsweise in den Zeiträumen, in denen Rampen bzw. schleichende Druckänderungen erfaßt werden. Der Innendruck wird aus der Differenz zwischen den Meßsignalen und der vorhergesagten Relaxation ermittelt.

Von der beschriebenen Vorgehensweise kann in vielerlei Hinsicht abgewichen werden, ohne den Kerngedanken der Erfindung zu verlassen.

Beispielsweise kann man die Optimierung stets zeitgesteuert starten, wobei die Zeitabstände zwischen den einzelnen Stützstellen differieren. Die ersten beiden Optimierungen werden dann mit sehr kurzen Abständen vorgenommen, um einen schnellen Meßbeginn zu ermöglichen. Dies bedeutet sehr kleine Zeitabstände der Stützstellen, um innerhalb eines Schieberegisters stets einen Teil alte und einen Teil neue Werte zur Anpassung des Modells zur Verfügung zu haben. Unterschiedliche Zeitabstände sind jedoch keine generelle Voraussetzung für das Verfahren. Sie haben allerdings den Nutzen, daß sie den Meßbeginn früher ermöglichen.

Es ist auch nicht zwingend erforderlich, die Stützstellen zeitgesteuert zu ermitteln und die Anpassung zeitgesteuert zu starten. Es muß lediglich sichergestellt werden, daß die Überprüfung bzw. Anpassung des Relaxationsverlaufs von Zeit zu Zeit verbessert wird, bzw. neue Stützstellen eingefügt werden. Die gemischte Vorgehensweise, d.h. zeitgesteuert einerseits und stützstellengesteuert andererseits, ist jedoch für einige Anwendungen vorteilhaft.

## Patentansprüche

1. Verfahren zur nichtinvasiven Innendruckmessung in elastischen Gefäßen, bei dem eine Kraft an der Mantelfläche des Gefäßes gemessen und der Innendruck mit Hilfe einer Differenz aus der gemessenen Kraft und einem im voraus abgeschätzten Relaxationsverlauf des Gefäßes ermittelt wird, **dadurch gekennzeichnet, daß** der Relaxationsverlauf nach Beginn der Messung wiederholt überprüft wird.

2. Verfahren nach Anspruch 1, **dadurch gekennzeichnet, daß** der Relaxationsverlauf mit Hilfe eines Mittelungsverfahrens ermittelt wird.

3. Verfahren nach Anspruch 2, **dadurch gekennzeichnet, daß** eine Mittelwertbildung auf mindestens zwei unterschiedliche Arten erfolgt, die sich durch ihre Glättungsbreite unterscheiden.

4. Verfahren nach Anspruch 3, **dadurch gekennzeichnet, daß** fortlaufend eine Differenz der Mittelwerte mit unterschiedlichen Glättungsbreiten gebildet wird.

5. Verfahren nach einem der Ansprüche 2 bis 4, **dadurch gekennzeichnet, daß** eine Periodizität der gemessenen Kraft ermittelt und eine Fensterbreite der Mittelwertbildung zumindest von Zeit zu Zeit auf die Periodizität abgestimmt wird.

**6.** Verfahren nach einem der Ansprüche 1 bis 5, **dadurch gekennzeichnet, daß** man fortlaufend eine erste Grenze bildet, die sich daraus ergibt, daß der Relaxationsverlauf monoton fällt, und eine zweite Grenze, die sich daraus ergibt, daß die Steigung des Relaxationsverlaufs abnimmt, und eine Veränderung des Innendrucks erkannt wird, wenn der Relaxationsverlauf eine der beiden Grenzen überschreitet.

**7.** Verfahren nach einem der Ansprüche 1 bis 6, **dadurch gekennzeichnet, daß** man zur Vorhersage des Relaxationsverlaufs wiederholt Stützstellen bestimmt.

**8.** Verfahren nach Anspruch 7, **dadurch gekennzeichnet, daß** die Stützstellen in eine Initialisierungsphase an vorgegebenen Zeitpunkten und in einer Meßphase nach einer vorbestimmten Änderung des vorhergesagten Relaxationsverlaufs ermittelt werden.

**9.** Verfahren nach Anspruch 7 oder 8, **dadurch gekennzeichnet, daß** Stützstellen nicht ermittelt werden, solange eine Änderung des Innendrucks erkannt wird.

**10.** Verfahren nach einem der Ansprüche 7 bis 9, **dadurch gekennzeichnet, daß** der Relaxationsverlauf anhand der Stützstellen mit Hilfe eines nichtlinearen Optimierungsverfahrens vorhergesagt wird.

**11.** Verfahren nach Anspruch 10, **dadurch gekennzeichnet, daß** die Vorhersage in der Initialisierungsphase stützstellengesteuert und in der Meßphase zeitgesteuert erfolgt.

**12.** Verfahren nach Anspruch 10 oder 11, **dadurch gekennzeichnet, daß** zur Optimierung eine vorbestimmte Anzahl der zuletzt ermittelten Stützstellen verwendetet wird.

**13.** Verfahren nach einem der Ansprüche 7 bis 12, **dadurch gekennzeichnet, daß** der Relaxationsverlauf anhand der Stützstellen mit Hilfe eines mathematischen Modells des Schlauches vorhergesagt wird.

**Claims**

**1.** Method for the non-invasive measurement of internal pressure in elastic vessels, in which method a force is measured at the outer surface of the vessel and the internal pressure is determined by forming a difference between the measured force and a previously estimated relaxation pattern of the vessel, **characterized in that** the relaxation pattern can be repeatedly verified after the beginning of measurement.

**2.** Method according to Claim 1, **characterized in that** the relaxation pattern is determined with the assistance of an averaging procedure.

**3.** Method according to Claim 2, **characterized in that** an average is calculated in at least two different ways, which differ from one another in their smoothing width.

**4.** Method according to Claim 3, **characterized in that** a difference is continuously formed between the averages with different smoothing widths.

**5.** Method according to one of Claims 2 to 4, **characterized in that** a periodicity of the measured force is ascertained and a window width of the averaging is at least intermittently matched to the periodicity.

**6.** Method according to one of Claims 1 to 5, **characterized in that** a first threshold is continuously formed, which derives from the fact that the relaxation pattern declines monotonously, and a second threshold is continuously formed, which derives from the fact that the gradient of the relaxation pattern decreases, and a change in the internal pressure is recognized when the relaxation pattern exceeds one of the two thresholds.

**7.** Method according to one of Claims 1 to 6, **characterized in that** support points are repeatedly identified to predict the relaxation pattern.

**8.** Method according to Claim 7, **characterized in that** the support points are determined in an initialization phase at predetermined points in time and in a measurement phase after a predetermined change in the predicted relaxation pattern.

**9.** Method according to Claim 7 or 8, **characterized in that** support points are not determined while a change in the internal pressure is recognized.

**10.** Method according to one of Claims 7 to 9, **characterized in that** the relaxation pattern is predicted by reference to the support points with the assistance of a non-linear optimization method.

**11.** Method according to Claim 10, **characterized in that** the prediction is controlled with reference to support points in the initialization phase and with reference to time in the measurement phase.

**12.** Method according to Claim 10 or 11, **characterized**

**in that** a predetermined number of the most recently determined support points are used for optimization.

13. Method according to one of Claims 7 to 12, **characterized in that** the relaxation method is predicted with reference to the support points using a mathematical model of the tube.

**Revendications**

1. Procédé de mesure de pression interne, non invasive, dans des contenants élastiques, selon lequel une force est mesurée sur la surface d'enveloppe du contenant et la pression interne est déterminée au moyen d'une différence formée à partir de la force mesurée et d'une évolution de relaxation du contenant estimée au préalable, **caractérisé en ce que** l'évolution de relaxation est contrôlée d'une manière répétée après le début de la mesure.

2. Procédé suivant la revendication 1, **caractérisé en ce que** l'évolution de relaxation est déterminée au moyen d'un procédé de formation de moyenne.

3. Procédé suivant la revendication 2, **caractérisé en ce qu'**une formation de valeur moyenne a lieu d'au moins de manières différentes qui différent par leur largeur d'égalisation.

4. Procédé suivant la revendication 3, **caractérisé en ce qu'**ensuite, une différence des valeurs moyennes comportant des largeurs d'égalisation différentes est formée.

5. Procédé suivant l'une des revendications 2 à 4, **caractérisé en ce qu'**une périodicité de la force mesurée est déterminée et une largeur de fenêtre de la formation de valeur moyenne est adaptée, au moins de temps à autre, à la la périodicité.

6. Procédé suivant l'une des revendications 1 à 5, **caractérisé en ce qu'**on forme ensuite une première limite, qui résulte du fait que l'évolution de relaxation décroît d'une manière monotone, et une seconde limite qui résulte du fait que la pente de l'évolution de relaxation diminue, et une variation de la pression intérieure est constatée lorsque l'évolution de relaxation dépasse vers le haut l'une des deux limites.

7. Procédé suivant l'une des revendications 1 à 6, **caractérisé en ce que**, pour prédire l'évolution de relaxation, on détermine d'une manière répétée des emplacements d'appui.

8. Procédé suivant la revendication 7, **caractérisé en ce que** les emplacement d'appui sont déterminés dans une phase d'initialisation à des instants préfixés et dans une phase de mesure après une variation prédéterminée de l'évolution de relaxation prédite.

9. Procédé suivant la revendication 7 ou 8, **caractérisé en ce que** des emplacements d'appui ne sont pas déterminés tant qu'une variation de la pression interne n'est pas constatée.

10. Procédé suivant l'une des revendications 7 à 9, **caractérisé en ce que** l'évolution de relaxation est prédite à l'aide des emplacements d'appui au moyen d'un procédé d'optimisation non linéaire.

11. Procédé suivant la revendication 10, **caractérisé en ce que** la prédiction a lieu dans la phase d'initialisation en étant commandée en emplacements d'appui et dans la phase de mesure en étant commandée en temps.

12. Procédé suivant la revendication 10 ou 11, **caractérisé en ce que**, pour l'optimisation, un nombre prédéterminé des emplacements d'appui déterminés en dernier est utilisé.

13. Procédé suivant l'une des revendications 7 à 12, **caractérisé en ce que** l'évolution de relaxation est prédite à l'aide des emplacements d'appui au moyen d'un modèle mathématique du tuyau souple.

Fig. 1

Werte

Zeit

T0   30 T1   25   10   20

Fig. 2

Fig. 3

Fig. 4

EP 1 173 739 B1

Fig. 5